# EUROPEAN PATENT SPECIFICATION

(11) **EP 2 774 604 B1**
(45) Date of publication and mention of the grant of the patent: **30.08.2017**
(21) Application number: 13158445.0
(22) Date of filing: 08.03.2013
(51) Int. Cl.: A61K 8/37, A61K 8/34, A61K 8/891, A61Q 5/02, A61K 8/35, A61Q 19/00, A61K 8/92, A61K 8/06

(54) **Cosmetic compositions**
Kosmetische Zubereitungen
Compositions cosmétiques

(43) Date of publication of application: 10.09.2014
(73) Proprietor: Symrise AG, 37603 Holzminden (DE)
(72) Inventor: Pesaro, Manuel, 37603 Holzminden (DE); Lange, Sabine, 37603 Holzminden (DE); Schmaus, Gerhard, 37671 Höxter (DE)
(74) Representative: Fabry, Bernd

(56) References cited:
- EP-A2- 1 681 046
- WO-A2-2007/124889
- WO-A2-2011/141110
- RAJABI L ET AL: "Acetophenones with selective antimycobacterial activity", LETTERS IN APPLIED MICROBIOLOGY, OXFORD, GB , vol. 40, no. 3 1 March 2005 (2005-03-01), pages 212-217, XP002693043, ISSN: 1472-765X, DOI: 10.1111/J.1472-765X.2005.01657.X Retrieved from the Internet: URL:http://onlinelibrary.wiley.com/doi/10. 1111/j.1472-765X.2005.01657.x/full [retrieved on 2013-02-18]

## Description

### Field of invention

The present invention belongs to the area of cosmetics and refers to uses of acetophenone derivatives in specific cosmetic compositions

### State of the art

Typically, cosmetic compositions comprise a multitude of components. Looking at the listing of ingredients for an average night cream one can find up to 20 positions and there has been a tendency to add as many ingredients as possible in order to address many different issues, all at the same time. As a matter of fact, many consumers equal complexity of a composition with benefit and are accepting high prices, since (s)he expect also high performance. On the other hand, the more complex a composition becomes, the more difficult it is to avoid negative interactions between the components. A major problem for cosmetic compositions, of course in particular for all types of emulsions, is still their stability, especially in case they are subjected to difficult storage conditions, like high or low temperatures. See e.g. WO2007124889. Another object is related to the sensorial profile. Spreadability, afterfeel and smell are important parameters contributing to the overall liking of a cosmetic formulation after application to skin:
The faster the oil bodies of an emulsion are spread on the skin the better is the perception of the customer. The spreading behaviour of a formulation - and therefore its sensorial profile - is linked to the average particle size of the droplets in the composition. The smaller the droplets are, the faster the spreading is. As a consequence, there is still a need for additives allowing to shift the average particle size distribution to lower values.

Another problem especially for shampoo compositions is foam stability and viscosity. See e.g. EP1681046. Therefore, the object of the present invention has been to identify a multi-functional additive for cosmetic formulations, which does not negatively interact with other ingredients while improving the stability, viscosity and the sensorial profile of the cosmetic compositions containing this additive.

### Description of the invention

Object of the present invention the use of an acetophenone derivative of formula(I) in a cosmetic composition, comprising
(a) at least one acetophenone derivative of formula (I) 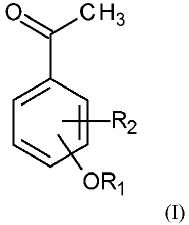 in which
   R₁ stands for hydrogen or methyl, and
   R₂ stands for hydrogen, hydroxyl or a -OCH₃ group,
   or a cosmetically or pharmaceutically acceptable salt thereof in a working amount of from 0.1 to 0.5 % b.w. - calculated on the topical composition,
(b) at least one oil body or wax, and/or
(c) at least one emulsifier and optionally
(d) at least one active principle,
   - to improve the stability of the cosmetic composition through reduction of the average particle size of droplets and
   - to improve the sensorial profile of the cosmetic formulations,
   - and to improve foam stability and increase viscosity of the cosmetic formulation which are shampoos.

Surprisingly it has been observed that the acetophenone derivatives according to the present invention serve all the above mentioned needs simultaneously:
▪ adjunction of acetophenone derivatives of formula (I) improves the stability of cosmetic formulations even under difficult storage conditions, and
▪ at the same time, adjunction of the acetophenone derivatives in a cosmetic formulation improve its sensorial profile. In particular, acetophenone derivatives lead to the formation of smaller droplets in the compositions. Due to the fact the oil or water droplets more finely divided, the sensation on skin is improved. For example, the greasy afterfeel of creams and lotions can be reduced in that way. The deodorants can also have a smoother feeling and a more creamy feeling when applied to the skin;
▪ furthermore, it has been observed that acetophenone derivatives of formula (I) improve the foam stability of shampoo and in addition, lead to increased viscosity of shampoo formulations.

Therefore, acetophenone derivatives of formula (I) serve the need for so-called "true multitasking ingredients".

### Acetophenone derivatives

Acetophenone derivatives used according to the present invention represent known compounds that can be obtained by ordinary methods of organic chemistry. It is understood that both substituents OR₁ and R₂ can be located in ortho, meta or para position towards the methyl-keto group.

Preferably, the species are selected from the group consisting of 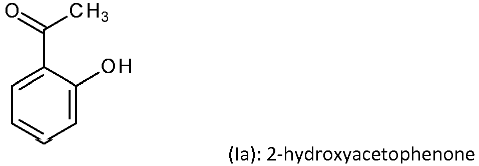 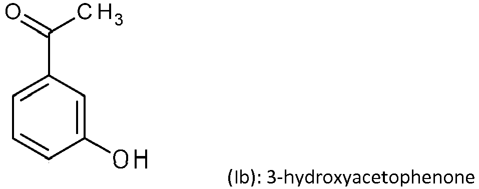 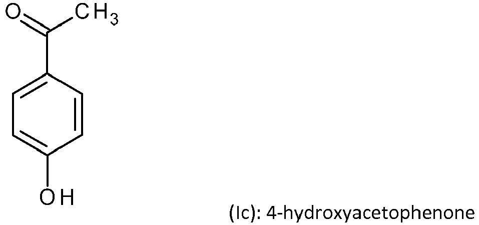 or their mixtures. In as far the definition refers to cosmetically or pharmaceutically acceptable salts of said derivatives, this means that these salts can be safely used for pharmaceutical purposes. This does not mean that the present invention or any aspect thereof is restricted to the use of a compound of formula (I) or a corresponding mixture for pharmaceutical purposes. Generally, if a salt can be used for pharmaceutical purposes it can likewise be used for cosmetic purposes, or in food or beverage formulations. In particular, the sodium and potassium and ammonium salts of compounds of formula (I) are considered as (pharmaceutically) acceptable salts. In some cases the utilization of the respective ionic compound or solvate carrier proves to be superior to the unmodified derivative. The (pharmaceutically) acceptable salts (and the corresponding solvates) of compounds of formula (I) can be prepared by standard procedures. Hereinafter, any reference to a compound of formula (I) or a corresponding mixture as defined above is to be understood as comprising an additional reference to corresponding (pharmaceutically) acceptable salts thereof.

In a preferred embodiment the preservative comprise or contain two or three of the compounds (Ia), (Ib) and (Ic), as for example 2-hydroxyacetophenone and 3- hydroxyacetophenone, or 2-hydroxyacetophenone and 4-hydroxyacetophenone, or 3-hydroxyacetophenone and 4-hydroxyacetophenone.

The composition contains the acetophenone derivatives in a working amount of from 0.1 to 0.5 % b.w. - calculated on the total composition.

### Oil bodies

Suitable oil bodies (component b1), are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.), aliphatic or naphthenic hydrocarbons, such as, for example, squalane, squalene or dialkylcyclohexanes, and/or mineral oils.

### Waxes

Among the group of suitable waxes (component b2) one can differentiate between the following types:
- superfatting agents
- consistency factors
- pearlising waxes, and
- natural waxes

***Superfatting agents.*** Superfatting agents may be selected from substances such as for example, lanolin and lecithin and also polyethoxylated or acylated lanolin and lecithin derivatives, polyol fatty acid esters, monoglycerides and fatty acid alkanolamides; fatty acid alkanolamides can also serve as foam stabilizers.

***Consistency factors.*** The consistency factors can be for example fatty alcohols or hydroxyfatty alcohols containing 12 to 22 and preferably 16 to 18 carbon atoms and also partial glycerides, fatty acids or hydroxyfatty acids of the same carbon range. A combination of these substances with alkyl oligoglucosides and/or fatty acid N-methyl glucamides of the same chain length and/or polyglycerol poly-12-hydroxystearates is preferably used.

***Pearlising waxes.*** Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

***Natural waxes.*** Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Emulsifiers

As an optional, the compositions according to the present invention may also include emulsifiers (component c). The emulsifiers may be of non-ionic, anionic, cationic and/or amphoteric nature.

In particular preferred are **non-ionic emulsifiers,** such as:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The most preferred emulsifiers are described in more detail as follows:

### C.1 Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### C.2 Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesqui-hydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### C.3 Polyglycerol esters

Typical examples of suitable polyglycerol esters are Polyglyceryl-2 Dipolyhydroxystearate (Dehymuls^{®} PGPH), Polyglycerin-3-Diisostearate (Lameform TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

### C.4 Anionic emulsifiers

Typical anionic emulsifiers are aliphatic C₁₂₋₂₂ fatty acids, such as palmitic acid, stearic acid or behenic acid for example, and C₁₂₋₂₂ dicarboxylic acids, such as aze laic acid or sebacic acid for example.

### C.5 Amphotheric or zwitterionic emulsifiers

Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Active principles

The compositions according to the present invention may contain additional ingredients (component d) encompassed by the term "active principles". Examples for suitable ingredients are abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, ant-statics, binders, buffers, carrier materials, chelating agents, cell stimulants, cleansing agents, care agents, depilatory agents, surface-active substances, deodorizing agents, antiperspirants, softeners, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, anti-corrosives, aromas, flavouring substances, odoriferous substances, polyols, surfactants, electrolytes, organic solvents or silicone derivatives and the like as additional auxiliaries and additives.

### D.1 Thickening agents and rheology additives

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### D.2 Polymers

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine , Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bisdimethylamino-1,3-propane, cationic guar gum such as, for example, Jaguar^{®}CBS, Jaguar C-17, Jaguar C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### D.3 Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### D.4 Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, it is advantageous to combine compounds of formula (I) with active ingredients which penetrate into the skin and protect the skin cells from inside against sunlight-induced damage and reduce the level of cutaneous matrix metalloproteases. Preferred respective ingredients, so called arylhydrocarbon receptor antagonists, are described in WO 2007/128723, incorporated herein by reference. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan^{®}HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150)

Broadband filters which are preferably combined with one or more compounds of formula (I) in a preparation according to the present invention are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl)propyl) (Mexoryl®XL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine

UV-A filters filters which are preferably combined with one or more compounds of formula (I) in a preparation according to the present invention are selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds in accordance with DE 100 55 940 A1 (= WO 2002 038537 A1)

UV filters which are more preferably combined with one or more compounds of formula (I) in a preparation according to the present invention are selected from the group consisting of
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCI name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds in accordance with DE 100 55 940 (= WO 02/38537).

Advantageous primary and also secondary sun protection factors are mentioned in WO 2005 123101 A1**.** Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) so that the formulation according to the invention has a light protection factor of greater than or equal to 2 (preferably greater than or equal to 5). Such formulations according to the invention are particularly suitable for protecting the skin and hair.

### D.5 Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂) zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### D.6 Actives modulating skin and/or hair pigmentation

Preferred active ingredients for skin and/or hair lightening are selected from the group consisting of:
kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates (preferably one or more cyclohexyl carbamates disclosed in WO 2010/122178 and WO 2010/097480), sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners as component (b) are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases") as described for example in EP 0 584 178, tetrasubstituted cyclohexene deriva-tives as described for example in WO 2005/032501 , isoprenoids as described in WO 2005/102252 and in WO 2006/010661 , melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, microalgae extracts, in particular Isochrysis galbana, trehalose, erythru-lose and dihydroxyacetone. Flavonoids which bring about skin and hair tinting or brown-ing (e.g. quercetin, rhamnetin, kaempferol, fisetin, genistein, daidzein, chrysin and api-genin, epicatechin, diosmin and diosmetin, morin, quercitrin, naringenin, hesperidin, phloridzin and phloretin) can also be used.

The amount of the aforementioned examples of additional active ingredients for the modulation of skin and hair pigmentation (one or more compounds) in the products according to the invention is then preferably 0.00001 to 30 wt.%, preferably 0.0001 to 20 wt.%, particularly preferably 0.001 to 5 wt.%, based on the total weight of the prepa-ration.

### D.7 Anti-ageing actives

In the context of the invention, anti-ageing or biogenic agents are, for example antioxidants, matrix-metalloproteinase inhibitrors (MMPI), skin moisturizing agents, glycosaminglycan stimulkators, anti-inflammatory agents, TRPV1 antagonists and plant extracts.
(i) **Antioxidants.** amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.
(ii) **Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonyl-fluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02 069992 A1 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1, incorporated herein by reference) as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.
(iii) **Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.
(iv) **Glycosaminoglycan stimulators.** Preferred compositions comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.
(v) **Anti-inflammatory agents.** The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1, are preferred anti-itch ingredients in a composition according to the present invention.
   Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.
(vi) **TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1, or indirect modulators of TRPV1 by an activation of the µ-receptor, e.g. acetyl tetrapeptide-15, are preferred.
(vii) **Anti-cellulite agents.** Anti-cellulite agents and lipolytic agents are preferably selected from the group consisting of those described in WO 2007/077541, and beta-adrenergic receptor agonists such as synephrine and its derivatives, and cyclohexyl carbamates described in WO 2010/097479. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.
(viii) **Fat enhancing agents.** Formulations and products according to the present invention may also comprise one or more fat enhancing and/or adipogenic agents as well as agents enhancing or boosting the activity of fat enhancing agents. A fat enhancing agent is for example hydroxymethoxyphenyl propyl-methylmethoxybenzofuran (trade name: Sym3D^{®}).

### D.8 Hair growth activators or inhibitors

Formulations and products according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, formulations and products according to the present invention may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### D.9 Cooling agents

The compositions may also contain one or more substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (I-menthoxy)-1,2-propandiol, (I-menthoxy)-2-methyl-1,2-propandiol, I-menthyl-methylether), menthylesters (for example menthylformiate, menthylacetate, menthylisobutyrate, menthyllactates, L-menthyl-L-lactate, L-menthyl-D-lactate, menthyl-(2-methoxy)acetate, menthyl-(2-methoxyethoxy-)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], as described in US 4,150,052, menthanecarboxylic acid-N-(4-cyanophenyl)amide or menthanecarboxylic acid-N-(4-cyanomethylphenyl)amide as described in WO 2005 049553 A1, methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates (preferably those described in EP 2033688 A2).

### D.10 Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### D.11 Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### D.12 Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### D.13 Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethyl-pentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-ylmethoxy-phenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon UD (protein/undecylenic acid condensate), zinc pyrithione, aluminium pyrithione and magnesium pyrithione/dipyrithione magnesium sulfate.

### D.14 Carriers and Hydrotropes

**Preferred cosmetics carrier** materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### D.15 Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### D.16 Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxy-citronellal, lilial and bourgeonal. Examples of suitable ketones are the ionones, ·-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### D.17 Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

### D.18 Preparations

Preferred compositions according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation.

### Industrial application

In a preferred embodiment the compositions as used according to the present invention comprise the components in the following amounts:
(a) acetophenone derivatives of formula (I) in a working amount of from 0.1 to 0.5 % b.w. - calculated on the total composition;
(b) about 99.9 to about 50, preferably about 95 to about 65 and more preferably about 80 to about 70 % b.w. oil bodies and/or waxes;
(c) 0.1 to about 25, preferably about 1 to about 15 and more preferably about 4 to about 8 % b.w. emulsifiers;
(d) 0 to about 25, preferably about 0.5 to about 10 and more preferably about 1 to about 5 % b.w. active principles;
on condition that the amounts add - optionally together with water and additional ingredients - to 100 % b.w.

The inventive compositions may contain water or are essentially free of water. Essentially free means that the amount of water is less than 2, preferably less than 1 and more preferably less than 0.5 % b.w. calculated on the final product.

The compositions according to the invention may represent o/w or w/o or multiple o/w/o or w/o/w emulsion. They can be used as an intermediate or a final product for example in the form of a lotion, a cream or a stick. As explained above the present invention relates to the use of acetophenone derivatives according to formula (I) as stabilizers for cosmetic compositions, in particular for reducing the average particle size of the droplets in an emulsion and as additive able to improve the sensorial profile of cosmetic compositions.

### Examples

### Examples 1 to 3, Comparative Example C1

O/W emulsions were prepared by heating a lipid phase A and an aqueous phase B separately to approximately 80°C. Then the aqueous phase B was added to the lipid phase A and mixed in an Ultra-Turrax for 2 minutes at 5.000 rpm. The emulsion thus obtained was allowed to cool down for 10 minutes using a vane stirrer at 150 rpm. Finally, the pH value was adjusted to about 8.0 by adding aqueous sodium hydroxide solution. The stability of the emulsions was determined using an analytical centrifuge of the LUMisizer^{®} type (L.U.M. GmbH) which measures the change in transmission over time. The instability index is defined between 0 (very stable) and 1 (complete separation). The experiments were conducted at 25 °C with a speed of 2.500 rpm over 1.25 h corresponding to a storage time of about 1 month. The composition of the emulsions and the stability results are provided in Table 1. Examples 1 to 3 are according to the invention, Example C1 serves for comparison.

**Table 1**

| O/W emulsions comprising Emulsiphos^{®} | | | | | |
|---|---|---|---|---|---|
| **Phase** | **Compound** | **C1** | **1** | **2** | **3** |
| **A** | Potassium Cetyl Phosphate (and) Hydrogenated Palm Glycerides | 2.0 | 2.0 | 2.0 | 2.0 |
| | **Emulsiphos^{®} PN 677660** | | | | |
| | Cetearyl Alcohol | 0.7 | 0.7 | 0.7 | 0.7 |
| | **Lanette^{®} O** | | | | |
| | Capric Caprylic Glycerides | 0.8 | 0.8 | 0.8 | 0.8 |
| | **Myritol^{®} 312** | | | | |
| | Cetearyl Ethylhexanoate | 4.0 | 4.0 | 4.0 | 4.0 |
| | **PCL Liquid 100** | | | | |
| | Dimethicone | 0.1 | 0.1 | 0.1 | 0.1 |
| | **Abil^{®} 350** | | | | |
| **B** | 4-Hydroxyactophenone | - | 0.1 | 0.3 | 0.5 |
| | Glycerol | 1.5 | 1.5 | 1.5 | 1.5 |
| | Water | Ad 100 | | | |
| ***Instability index (mean of 3 experiments)*** | | 0.35 | 0.30 | 0.29 | 0.29 |
| ***Statistical significance versus C1 (T-Test)¹⁾*** | | | n.d. | 0,004 | 0,027 |

| | | | | | |
|---|---|---|---|---|---|
| n.d. = not determined *¹⁾* values below 0.05 indicate statistical significance | | | | | |

The examples and comparative examples clearly demonstrate that adding 4-hydroxyacetophenone to the emulsions leads to significantly less separation and improves the stability of the emulsion.

### Examples 4 to 6, Comparative Example C2

O/W emulsions were prepared according to the procedure explained for Examples 1 to 3 with the only exception that the pH value was adjusted to about 9.1. The stability of the emulsions was again determined using an analytical centrifuge of the LUMisizer^{®} type (L.U.M. GmbH). The composition of the emulsions and the stability results are provided in **Table 2.** Examples 4 to 6 are according to the invention, Example C2 serves for comparison.

**Table 2**

| O/W emulsions comprising Dracorin^{®} | | | | | |
|---|---|---|---|---|---|
| **Phase** | **Compound** | **C2** | **4** | **5** | **6** |
| **A** | Glyceryl Stearate Citrate | 2.0 | 2.0 | 2.0 | 2.0 |
| | **Dracorin^{®} CE** | | | | |
| | Cetearyl Alcohol | 0.2 | 0.2 | 0.2 | 0.2 |
| | **Lanette^{®} O** | | | | |
| | Capric Caprylic Glycerides | 10.0 | 10.0 | 10.0 | 10.0 |
| | **Myritol**^{®} **312** | | | | |
| | Avocado oil | 2.0 | 2.0 | 2.0 | 2.0 |
| | Dimethicone | 0.1 | 0.1 | 0.1 | 0.1 |
| | **Abil**^{®} **350** | | | | |
| **B** | 4-Hydroxyacetophenone | - | 0.1 | 0.3 | 0.5 |
| | Glycerol | 1.5 | 1.5 | 1.5 | 1.5 |
| | Water | Ad 100 | | | |
| ***Instability index (mean of 3 experiments)*** | | 0.37 | 0.31 | 0.30 | 0.26 |
| ***Statistical significance versus C2 (T-Test)¹⁾*** | | | n.d. | 0,062 | 0,013 |

| | | | | | |
|---|---|---|---|---|---|
| n.d. = not determined *¹⁾* values below 0.05 indicate statistical significance | | | | | |

Also the second set of examples and comparative examples clearly demonstrates that adding 4-hydroxyacetophenone to the emulsions leads to significantly less separation and improves the emulsion stability.

### Examples 1 to 6, Comparative Examples C1 and C2

O/W emulsions were prepared according to the procedure explained for Examples 1 to 3. The particle size distributions of the emulsions according to Example 1 to 6 and Comparative Examples C1 and C2 were measured using a Mastersizer Micro (Malvern) using the principle of laser diffraction. The results are shown in **Table 3.**

**Table 3**

| Particle size distribution (all values in µm) | | | | | | | | |
|---|---|---|---|---|---|---|---|---|
| **Particle Size** | **C1** | **2** | **3** | **4** | **C2** | **4** | **5** | **6** |
| D (v, 0.1) = 10 % | 2.10 | 1.98 | 1.76 | 1.83 | 1.64 | 1.42 | 1.45 | 1.55 |
| D (v, 0.5) = 50 % | 11.32 | 10.42 | 8.93 | 9.40 | 10.93 | 7.31 | 7.59 | 8.81 |
| D (v, 0.9) = 90 % | 33.73 | 28.37 | 18.7 | 23.76 | 20.35 | 13.64 | 14.20 | 17.50 |

The index "v" refers to a volume based particle size distribution; all values were taken from the sum distribution Qᵣ. For example, the value 1.64 for C2 means that 10 % of the particles show a particle size less than 1.64 µm and so on.

As one can see, adding of 4-hydroxyacetophenone shifts the maximum average particle size to smaller values and leads to more finely divided emulsions.

## Claims

1. Use of acetophenone derivative(s) of formula (I)
• to improve the stability of the cosmetic composition through reduction of the average particle size of droplets;
and
• to improve the sensorial profile of the cosmetic formulations;
and
• to improve foam stability and increase viscosity of the cosmetic formulation which are shampoos in a
cosmetic composition, comprising
(a) at least one acetophenone derivative of formula (I) 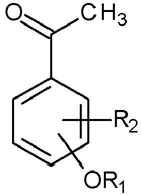 in which
R₁ stands for hydrogen or methyl, and
R₂ stands for hydrogen, hydroxyl or a -OCH₃ group,
or a cosmetically or pharmaceutically acceptable salt thereof in a working amount of from 0.1 to 0.5 % b.w. - calculated on the total composition,
(b) at least one oil body or wax, and/or
(c) at least one emulsifier and optionally
(d) at least one active principle,

2. The use of Claim 1, wherein the acetophenone derivatives are selected from the group consisting of 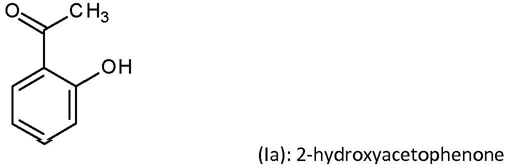 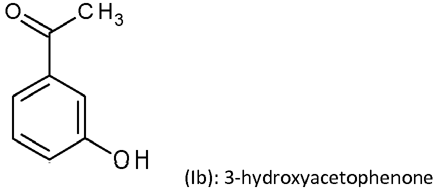 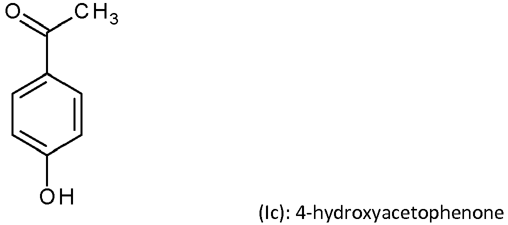 or their mixtures.

3. The use of Claim 2 comprising or consisting of two or three of the compounds (Ia), (Ib) and (Ic).

4. The use of Claim 1, wherein the acetophenone derivatives are present in amounts of from 0.1 to 0.5 % b.w. -calculated on the final composition.

5. The use of Claim 1, wherein the oil bodies (component b1) are selected from the group consisting of Guerbet alcohols based on fatty alcohols having 6 to 18 carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, esters of linear C₆-C₂₂-fatty acids with branched alcohols, esters of C₁₈-C₃₈- alkylhydroxy carboxylic acids with linear or branched C₆-C₂₂-fatty alcohols, esters of linear and/or branched fatty acids with polyhydric alcohols and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, Guerbet carbonates, based on fatty alcohols having 6 to 18, preferably 8 to 10, carbon atoms, esters of benzoic acid with linear and/or branched C₆-C₂₂-alcohols, linear or branched, symmetrical or asymmetrical dialkyl ethers having 6 to 22 carbon atoms per alkyl group, ring-opening products of epoxidized fatty acid esters with polyols, silicone oils and/or aliphatic or naphthenic hydrocarbons, mineral oils and their mixtures.

6. The use of Claim 1, wherein the waxes (component b2) are selected from the group consisting of super fatting agents, consistency factors, pearlising waxes, natural waxes and their mixtures.

7. The use of Claim 1, wherein the emulsifiers (component c) are selected from the group consisting of non-ionic, anionic, cationic, or amphoteric emulsifiers and their mixtures.

8. The use of Claim 1, wherein the active principles (component d) are selected from the group consisting of abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, ant-statics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, anti-corrosives, aromas, flavouring substances, odoriferous substances, polyols, surfactants, electrolytes, organic solvents or silicone derivatives

9. The use of Claim 1, wherein the cosmetic composition comprise the components in the following amounts:
(a) 0.1 to 0.5 % b.w. acetophenone derivatives of formula (I);
(b) about 99.9 to about 50 % b.w. oil bodies and/or waxes;
(c) 0.1 to about 25 % b.w. emulsifiers;
(d) 0 to about 25 % b.w. active principles;
on condition that the amounts add - optionally together with water and additional ingredients-to 100 % b.w.

10. The use of Claim 1, wherein the product either contains water or is essentially free of water

11. The use of Claim 1, wherein the product is an o/w or w/o or multiple o/w/o or w/o/w emulsion

12. The use of Claim 1, wherein the product is a lotion, a cream or a stick.

13. A method of stabilizing an emulsion against separation whereby a working amount from 0.1 to 0.5 % b.w. of 4-hydroxyacetophenone is added to a composition comprising
(b) at least one oil body or wax, and/or
(c) at least one emulsifier and optionally
(d) at least one active principle,
- calculated on the total emulsion.

14. Use of 4-hydroxyacetophenone as stabilizer against separation for cosmetic emulsion compositions comprising
(a) at least one acetophenone derivative of formula (I)
(b) at least one oil body or wax, and/or
(c) at least one emulsifier and optionally
(d) at least one active principle,
- calculated on the total emulsion.

15. A method of reducing the average particle size of the droplets in an emulsion whereby a working amount from 0.1 to 0.5 % b.w. of at least one acetophenone derivative of formula (I) is added to a composition comprising
(b) at least one oil body or wax, and/or
(c) at least one emulsifier and optionally
(d) at least one active principle,
- calculated on the total emulsion.

16. A method of improving foam stability and increasing viscosity of shampoos whereby a working
amount from 0.1 to 0.5 % b.w. of at least one acetophenone derivative of formula (I) is added to a composition comprising
(b) at least one oil body or wax, and/or
(c) at least one emulsifier and optionally
(d) at least one active principle,
- calculated on the total emulsion.

## Patentansprüche

1. Verwendung von Acetophenon Derivat(en) der Formel (I)
• um die Stabilität der kosmetischen Zusammensetzung durch die Reduzierung der durchschnittlichen Partikelgröße der Tröpfchen zu verbessern;
und
• um das sensorische Profil der kosmetischen Formulierungen zu verbessern; und
• um die Schaumstabilität zu verbessern und die Viskosität der kosmetischen Formulierung, welche Shampoos sind, zu erhöhen
in einer kosmetischen Zusammensetzung, umfassend
(a) mindestens ein Acetophenon Derivat der Formel (I) 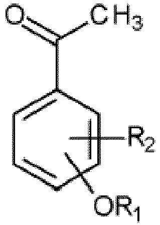 in welcher
R1 für Wasserstoff oder Methyl steht, und
R2 für Wasserstoff. Hydroxyl oder eine -OCH3 Gruppe steht,
oder einem kosmetisch oder pharmazeutisch akzeptablem Salz davon, in einer Arbeitsmenge von 0.1 bis 0.5 Gew.-% - bezogen auf die gesamte Zusammensetzung,
(b) mindestend ein Ölkörper oder Wachs, und/oder
(c) mindestens ein Emulgator und optional
(d) mindestens ein Wirkstoff.

2. Verwendung nach Anspruch 1, wobei die Acetophenon Derivate ausgewählt sind aus der Gruppe bestehend aus 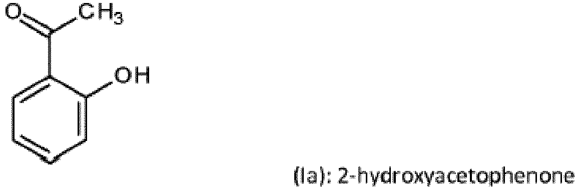 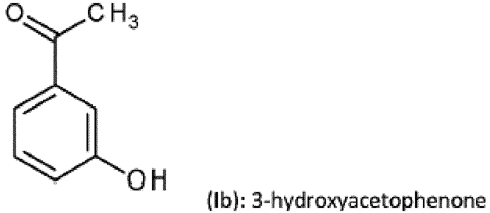 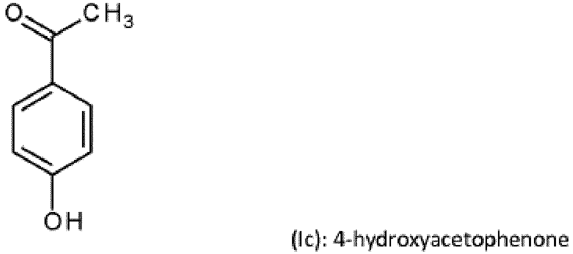 oder ihren Mischungen.

3. Verwendung nach Anspruch 2, umfassend oder bestehend aus zwei oder drei der Komponenten (Ia), (Ib) und (Ic).

4. Verwendung nach Anspruch 1, wobei die Acetophenon Derivate in einer Menge von 0,1 bis 0,5 Gew.-% vorliegen - bezogen auf die finale Zusammensetzung.

5. Verwendung nach Anspruch 1, wobei die Ölkörper (Komponente b1) ausgewählt sind aus der Gruppe bestehend aus Guerbet Alkoholen basierend auf Fettalkoholen mit 6 bis 18 Kohlenstoffatomen, Estern von linearen C₆-C₂₂ Fettsäuren mit linearen oder verzweigten C₆-C₂₂ Fettalkoholen oder Estern von verzweigten C₆-C₁₃ Carbonsäuren mit linearen oder verzweigten C₆-C₂₂ Fettalkoholen, Ester von linearen C₆-C₂₂ Fettsäuren mit verzweigten Alkoholen, Ester von C₁₈-C₃₈ Alkylhydroxycarbonsäuren mit linearen oder verzweigten C₆-C₂₂ Fettalkoholen, Ester von linearen und/oder verzweigten Fettsäuren mit mehrwertigen Alkoholen und/oder Guerbet Alkholen, Triglyceride basierend auf C₆-C₁₀ Fettsäuren, flüssige Mono-/Di-/Triglycerid Mischungen basierend auf C₆-C₁₈ Fettsäuren. Ester von C₆-C₂₂ Fettalkoholen und/oder Guerbet Alkoholen mit aromatischen Carbonsäuren, Ester von C₂-C₁₂ Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, vegetarische Öle, verzweigte primäre Alkohole, substituierte Cyclohexane, lineare und verzweigte C6-C22 Fettalkoholcarbonaten, Guerbet Carbonaten, basieren auf Fettalkoholen mit 6 bis 18, vorzugsweise 8 bis 10 Kohlenstoffastomen, Estern von Benzoesäure mit linear und/oder verzweigten C6-C22 Alkoholen, lineare oder verzweigte, symmetrische oder asymmetrische Dialkylether mit 6 bis 22 Kohlenstoffatomen mit Polyolen, Silikonölen und/oder aliphatischen oder naphtenischen Kohlenwasserstoffen, Mineralölen und ihren Mischungen.

6. Verwendung nach Anspruch 1, wobei due Wachse (Komponente b2) ausgewählt sind aus der Gruppe bestehend aus Super-Fettungsmitteln, Konsistenzfaktoren, Perlglanzwachsen, Naturwachsen und ihren Mischungen.

7. Verwendung nach Anspruch 1, wobei die Emulgatoren (Komponente c) ausgewählt sind aus der Gruppe bestehen aus nicht-ionischen, anionischen, kationischen oder amphoteren Emulgatoren und ihren Mischungen.

8. Verwendung nach Anspruch 1, wobei die Wirkstoffe (Komponente c) ausgewählt sind aus der Gruppe bestehend aus Schleifmittel, Anti-Akne-Mittel, Mittel gegen Alterung der Haut, Anti-Cellulitis-Mittel, Antischuppenmittel, entzündungshemmende Mittel, irritationsverhindernde Mittel, irritationshemmende Mittel, Antioxidantien, Adstringentien, schweißhemmende Mittel, antiseptische Mittel, Antistatika, Bindemittel, Puffer, Trägermaterialien, Chelatbildner, Zellstimulantien, Reinigungsmittel, Pflegemittel, Enthaarungsmittel, oberflächenaktive Substanzen, Desodorierungsmittel, Antiperspirantien, Weichmacher, Emulgatoren, Enzyme, ätherische Öle, Fasern, filmbildende Mittel , Fixiermittel, Schaumbildner, Schaumstabilisatoren, Stoffe zur Vermeidung von Schaumbildung, Schaumverstärker, Geliermittel, Gelbildner, Haarpflegemittel, Haarfärbemittel, Haarglättungsmittel, feuchtigkeitsspendende Mittel, feuchtigkeitsspendende Stoffe, Feuchtigkeit -Entwickler, Bleichmittel, Verstärkungsmittel, Fleckenentfernungsmittel, optisch aufhellende Mittel, Imprägniermittel, schmutzabweisende Mittel, Reibungsverminderungsmittel, Schmierstoffe, feuchtigkeitsspendende Cremes, Salben, Trübungsmittel, Weichmacher, Abdeckmittel, Poliermittel, Glanzmittel, Polymere, Pulver, Proteine, Nachöler, Abriebmittel, Silikone, Hautberuhigungsmittel, Hautreinigungsmittel, Hautpflegemittel , Hautpflegemittel, Hautaufhellungsmittel, Hautschutzmittel, Hautenthärter, Haarfördermittel, Kühlmittel, Hautkühlmittel, Erwärmungsmittel, Hautwärme, Stabilisatoren, UV-absorbierende Mittel, UV-Filter , Detergenzien, Stoffkonditioniermittel, Suspensionsmittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, Farbschutzmittel, Pigmente, Korrosionsschutzmittel, Aromen, Aromastoffe, Riechstoffe, Polyole, Tenside, Elektrolyte, organische Lösungsmittel oder Silikonderivate

9. Verwendung nach Anspruch 1, wobei die kosmetische Zusammensetzung die Komponenten in den folgenden Mengen umfasst:
(a) 0,1 bis 0,5 Gew.-% Acetophenon Derivate der Formel (I);
(b) ungefähr 99,9 bis ungefähr 50 Gew.-% Ölkörper und/oder Wachse;
(c) 0,1 bis ungefähr 25 Gew.-% Emulgatoren;
(d) 0 bis ungefähr 25 Gew.-% Wirkstoffe;
mit der Maßgabe, dass sich die Mengenangaben, optional zusammen mit Wasser und zusätzlichen Inhaltstoffen-zu 100 Gew.-% ergänzen.

10. Verwendung nach Anspruch 1, wobei das Produkt entweder Wasser enthält oder im Wesentlichen wasserfrei ist.

11. Verwendung nach Anspruch 1, wobei das Produkt eine o/w oder w/o oder eine mehrfach o/w/o oder w/o/w Emulsion ist

12. Verwendung nach Anspruch 1, wobei das Produkt eine Lotion, eine Creme oder ein Stick ist.

13. Verfahren zum Stabilisieren einer Emulsion gegen Separierung, wobei eine Arbeitsmenge von 0,1 bis 0,5 Gew.-% von 4-Hydroxyacetophenone zu einer Zusammensetzung gegeben wird, die Zusammensetzung umfassend
(b) mindestens ein Körperöl oder Wachs, und/oder
(c) mindestens einen Emulgator und optional
(d) mindestens ein Wirkstoff,
- bezogen auf die gesamte Emulsion.

14. Verwendung von 4-Hydroxyacetophenon als Stabilisator gegen Separierung von kosmetischen Emulsion Zusammensetzungen umfassen
(a) mindestens ein Acetophenon Derivat der Formel (I)
(b) mindestens ein Körperöl oder Wachs, und/oder
(c) mindestens ein Emulgator und optional
(d) mindestens ein Wirkstoff,
- bezogen auf die gesamte Emulsion.

15. Verfahren zur Reduzierung der durchschnittlichen Partikelgröße der Tröpfchen in einer Emulsion, wobei eine Arbeitsmenge von 0,1 bis 0,5 Gew.-% von mindestens einem Acetophenon Derivat der Formel (I) einer Zusammensetzung zugegeben wird, die Zusammensetzung umfassend
(b) mindestens ein Körperöl oder Wachs, und/oder
(c) mindestens ein Emulgator und optional
(d) mindestens ein Wirkstoff,
- bezogen auf die gesamte Emulsion.

16. Verfahren zur Verbesserung der Schaumstabilität und zur Erhöhung der Viskosität von Shampoos, wobei eine Arbeitsmenge von 0,1 bis 0,5 Gew.-% von mindestens einem Acetophenon Derivat der Formel (I) einer Zusammensetzung zugegeben wird, die Zusammensetzung umfassend
(b) mindestens ein Körperöl oder Wachs, und/oder
(c) mindestens ein Emulgator und optional
(d) mindestens ein Wirkstoff,
- bezogen auf die gesamte Emulsion.

## Revendications

1. Utilisation d'un ou plusieurs dérivés d'acétophénone de formule (I)
- afin d'améliorer la stabilité de la composition cosmétique par la réduction de la taille moyenne de particules de gouttelettes ; et
- afin d'améliorer le profil sensoriel des formulations cosmétiques ; et
- afin d'améliorer la stabilité de mousse et augmenter la viscosité de la formulation cosmétique qui sont des shampooings dans une composition cosmétique, comprenant
(a) au moins un dérivé d'acétophénone de formule (I) 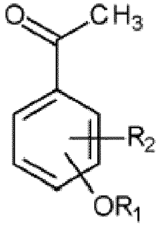 où
R1 représente hydrogène ou méthyle, et
R2 représente hydrogène, hydroxyle, ou un groupement OCH3,
ou un sel cosmétiquement ou pharmaceutiquement acceptable de celui-ci selon une quantité efficace allant de 0,1 à 0,5% en poids, calculé sur la base de la composition totale,
(b) au moins un corps huileux ou une cire, et/ou
(c) au moins un émulsifiant, et éventuellement
(d) au moins un principe actif.

2. Utilisation selon la revendication 1, dans laquelle les dérivés d'acétophénone sont choisis dans le groupe constitué par 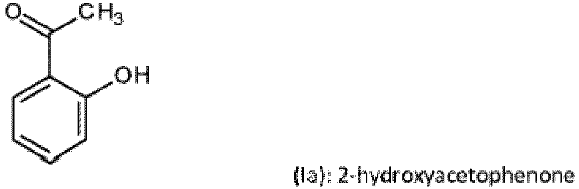 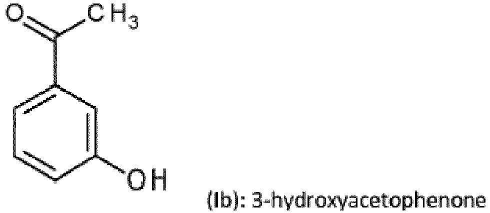 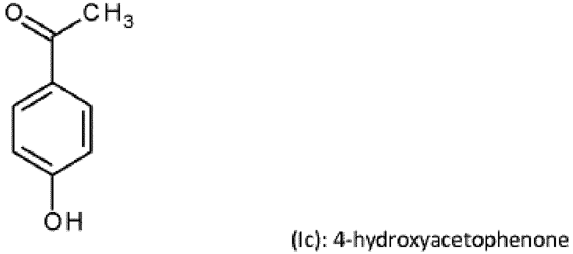 ou leurs mélanges.

3. Utilisation selon la revendication 2, comprenant ou consistant en deux ou trois parmi les composés (Ia), (Ib) et (Ic).

4. Utilisation selon la revendication 1, dans laquelle les dérivés d'acétophénone sont présents selon des quantités allant de 0,1 à 0,5% en poids, calculé sur la base de la composition finale.

5. Utilisation selon la revendication 1, dans laquelle les corps huileux (composant b1) sont choisis dans le groupe constitué par les alcools de Guerbet à base d'alcools gras ayant de 6 à 18 atomes de carbone, les esters d'acides gras en C6-C22 linéaires avec des alcools gras en C6-C22 linéaires ou ramifiés, ou les esters d'acides carboxyliques en C6-C13 ramifiés avec des alcools gras en C6-C22 linéaires ou ramifiés, les esters d'acides gras en C6-C22 linéaires avec des alcools ramifiés, les esters d'acides C18-C38-alkylhydroxy carboxyliques avec des alcools gras en C6-C22 linéaires ou ramifiés, les esters d'acides gras linéaires et/ou ramifiés avec des alcools polyhydriques et/ou des alcools de Guerbet, les triglycérides à base d'acides gras en C6-C10, les mélanges de mono-/di-/triglycérides liquides à base d'acides gras en C6-C18, les esters d'alcools gras en C6-C22 et/ou d'alcools de Guerbet avec des acides carboxyliques aromatiques, les esters d'acides dicarboxyliques en C2-C12 avec des alcools linéaires ou ramifiés ayant de 1 à 22 atomes de carbone ou des polyols ayant de 2 à 10 atomes de carbone et de 2 à 6 groupements hydroxyle, les huiles végétales, les alcools primaires ramifiés, les cyclohexanes substitués, les carbonates d'alcools gras en C6-C22 linéaires et ramifiés, les carbonates de Guerbet, à base d'alcools gras ayant de 6 à 18, préférablement de 8 à 10, atomes de carbone, les esters d'acide benzoïque avec des alcools en C6-C22 linéaires et/ou ramifiés, les éthers de dialkyle linéaires ou ramifiés, symétriques ou asymétriques, ayant de 6 à 22 atomes de carbone par groupement alkyle, les produits d'ouverture de cycle d'esters d'acides gras époxydés avec des polyols, les huiles de silicone et/ou les hydrocarbures naphténiques ou aliphatiques, les huiles minérales et leurs mélanges.

6. Utilisation selon la revendication 1, dans laquelle les cires (composant b2) sont choisies dans le groupe constitué par les agents surgraissants, les facteurs de consistance, les cires nacrantes, les cires naturelles et leurs mélanges.

7. Utilisation selon la revendication 1, dans laquelle les émulsifiants (composant c) sont choisis dans le groupe constitué par les émulsifiants non ioniques, anioniques, cationiques, ou amphotères, et leurs mélanges.

8. Utilisation selon la revendication 1, dans laquelle les principes actifs (composant d) sont choisis dans le groupe constitué par les abrasifs, les agents anti-acné, les agents antivieillissement de la peau, les agents anticellulite, les agents antipelliculaires, les agents anti-inflammatoires, les agents prévenant les irritations, les agents inhibant les irritations, les antioxydants, les astringents, les agents inhibant la transpiration, les agents antiseptiques, les antistatiques, les liants, les tampons, les matériaux véhicules, les agents chélateurs, les stimulants cellulaires, les agents nettoyants, les agents de soin, les agents épilatoires, les substances tensioactives, les agents désodorisants, les antitranspirants, les assouplissants, les émulsifiants, les enzymes, les huiles essentielles, les fibres, les agents filmogènes, les fixateurs, les agents moussants, les stabilisateurs de mousse, les substances anti-mousse, les renforçateurs de mousse, les agents gélifiants, les agents formateurs de gel, les agents de soin des cheveux, les agents de fixation des cheveux, les agents de lissage des cheveux, les agents apportant de l'humidité, les substances hydratantes, les substances retenant l'humidité, les agents de blanchiment, les agents renforçants, les agents détachants, les azurants optiques, les agents d'imprégnation, les agents résistant à la crasse, les agents de réduction de friction, les lubrifiants, les crèmes hydratantes, les onguents, les agents opacifiants, les agents plastifiants, les agents couvrants, les vernis, les agents de brillance, les polymères, les poudres, les protéines, les agents de re-huilage, les agents d'abrasion, les silicones, les agents d'apaisement de la peau, les agents de nettoyage de la peau, les agents de soin de la peau, les agents de cicatrisation de la peau, les agents d'éclaircissement de la peau, les agents de protection de la peau, les agents d'assouplissement de la peau, les agents de promotion des cheveux, les agents refroidissants, les agents de refroidissement de la peau, les agents chauffants, les agents de réchauffement de la peau, les stabilisants, les agents anti-UV, les filtres anti-UV, les détergents, les agents de conditionnement du linge, les agents de suspension, les agents de bronzage de la peau, les épaississants, les vitamines, les huiles, les cires, les matières grasses, les phospholipides, les acides gras saturés, les acides gras mono- ou polyinsaturés, les acides α-hydroxylés, les acides gras polyhydroxylés, les agents liquéfacteurs, les colorants, les agents de protection des couleurs, les pigments, les agents anti-corrosion, les arômes, les substances aromatisantes, les substances odorantes, les polyols, les agents tensioactifs, les électrolytes, les solvants organiques ou les dérivés de silicone.

9. Utilisation selon la revendication 1, dans laquelle la composition cosmétique comprend les composants selon les quantités suivantes :
(a) de 0,1 à 0,5% en poids de dérivés d'acétophénone de formule (I) ;
(b) d'environ 99,9 à environ 50% en poids de corps huileux et/ou de cires ;
(c) de 0,1 à environ 25% en poids d'émulsifiants ;
(d) de 0 à environ 25% en poids de principes actifs ;
à condition que les quantités totalisent - éventuellement conjointement avec de l'eau et des ingrédients supplémentaires -100% en poids.

10. Utilisation selon la revendication 1, dans laquelle le produit contient de l'eau ou bien est essentiellement dépourvu d'eau.

11. Utilisation selon la revendication 1, dans laquelle le produit est une émulsion h/e ou e/h ou multiple h/e/h ou e/h/e.

12. Utilisation selon la revendication 1, dans laquelle le produit est une lotion, une crème ou un bâtonnet.

13. Méthode de stabilisation d'une émulsion contre une séparation, où une quantité efficace allant de 0,1 à 0,5% en poids de 4-hydroxyacétophénone est ajoutée à une composition comprenant
(b) au moins un corps huileux ou une cire, et/ou
(c) au moins un émulsifiant, et éventuellement
(d) au moins un principe actif,
calculé sur la base de l'émulsion totale.

14. Utilisation de 4-hydroxyacétophénone comme stabilisant contre une séparation pour des compositions cosmétiques en émulsion, comprenant
(a) au moins un dérivé d'acétophénone de formule (I),
(b) au moins un corps huileux ou une cire, et/ou
(c) au moins un émulsifiant, et éventuellement
(d) au moins un principe actif,
calculé sur la base de l'émulsion totale.

15. Méthode de réduction de la taille moyenne de particules de gouttelettes dans une émulsion, où une quantité efficace allant de 0,1 à 0,5% en poids d'au moins un dérivé d'acétophénone de formule (I) est ajoutée à une composition comprenant
(b) au moins un corps huileux ou une cire, et/ou
(c) au moins un émulsifiant, et éventuellement
(d) au moins un principe actif,
calculé sur la base de l'émulsion totale.

16. Méthode d'amélioration de la stabilité de mouse et d'augmentation de la viscosité de shampooings, où une quantité efficace allant de 0,1 à 0,5% en poids d'au moins un dérivé d'acétophénone de formule (I) est ajoutée à une composition comprenant
(b) au moins un corps huileux ou une cire, et/ou
(c) au moins un émulsifiant, et éventuellement
(d) au moins un principe actif,
calculé sur la base de l'émulsion totale.
